# FASCICULE DE BREVET EUROPEEN

(11) **EP 1 028 120 B1**
(45) Date de publication et mention de la délivrance du brevet: **19.03.2003**
(21) Numéro de dépôt: 00400297.8
(22) Date de dépôt: 03.02.2000
(51) Int. Cl.: C07F 7/08, C07F 7/21, A61K 7/42, C08G 77/388

(54) **Nouveaux dérivés siliciés de benzimidazole-benzazoles N-substitués ou de benzofuranyl-benzazoles filtres, compositions cosmétiques photoprotectrices les contenant et utilisations**
Organosiliziumverbindungen mit N-substituierter Benzimidazol-Benzazolgruppe oder Benzofuranyl-Benzazolgruppe, diese enthaltende Sonnenschutzzusammensetzungen, und deren Verwendungen
Organosilicon compounds with N-substituted benzimidazole-benzazole or benzofuranyl-benzazole groups, sunscreen compositions containing these compounds and their use

(30) Priorité: 12.02.1999 FR 9901734
(43) Date de publication de la demande: 16.08.2000
(73) Titulaire: L'OREAL, 75008 Paris (FR)
(72) Inventeur: Richard, Hervé, 93420 Villepinte (FR); Luppi, Bernadette, 77290 Mitry Mory (FR)
(74) Mandataire: Miszputen, Laurent

(56) Documents cités:
- EP-A- 0 843 995

## Description

L'invention concerne de nouveaux dérivés siliciés de Benzimidazolyl-Benzazoles N-substitués ou de Benzofuranyl-Benzazoles, liposolubles, photostables et présentant un excellent pouvoir d'absorption dans le domaine des rayonnements UV. L'invention concerne également des compositions, notamment cosmétiques, contenant ces nouveaux dérivés, qui peuvent être destinées à la photoprotection de la peau et/ou des cheveux contre le rayonnement UV, en particulier le rayonnement solaire.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande croissante de moyens de contrôle de ce bronzage naturel afin de contrôler ainsi la couleur de leur peau. Il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

De nombreux composés destinés à la photoprotection (UV-A et/ou UV-B) de la peau ont été proposés à ce jour.

La plupart d'entre eux sont des composés aromatiques présentant une absorption des rayons UV dans la zone comprise entre 280 et 315 nm, ou dans la zone comprise entre 315 et 400 nm ou bien encore dans l'ensemble de ces deux zones. Ils sont le plus souvent formulés dans des compositions antisolaires qui se présentent sous la forme d'une émulsion de type huile-dans-eau (c'est à dire un support cosmétiquement acceptable constitué d'une phase continue dispersante aqueuse et d'une phase discontinue dispersée huileuse) et qui contiennent donc, à des concentrations diverses, un ou plusieurs filtres organiques classiques à fonction aromatique, lipophiles et/ou hydrophiles, capables d'absorber sélectivement les rayonnements UV nocifs, ces filtres (et leurs quantités) étant sélectionnés en fonction du facteur de protection solaire recherché (le facteur de protection solaire s'exprimant mathématiquement par le rapport du temps d'irradiation nécessaire pour atteindre le seuil érythématogène avec le filtre UV au temps nécessaire pour atteindre le seuil érythématogène sans filtre UV).

Outre leur pouvoir filtrant, ces composés à activité anti-UV doivent également présenter de bonnes propriétés cosmétiques dans les compositions qui les contiennent, une bonne solubilité dans les solvants usuels et en particulier les corps gras tels que les huiles et les graisses, ainsi qu'une bonne résistance à l'eau et à la transpiration (rémanence).

Parmi tous les composés aromatiques qui ont été proposés à cet effet, on peut citer les composés de Benzimidazolyl-Benzazoles N-substitués de la demande de brevet EP-A 0843995 ou les composés de Benzofuranyl-Benzoxazoles de la demande de brevet EP-A 0722714. La solubilité de ces molécules dans différents types de formulations utilisés en matière de protection solaire reste encore insuffisante.

La Demanderesse a découvert de manière surprenante de nouveaux dérivés siliciés de Benzimidazolyl-Benzazoles N-substitués ou de Benzofuranyl-Benzazoles présentant des propriétés améliorées, notamment au niveau de leur solubilité dans les corps gras et de leur stabilité à la lumière.

Plus précisément encore, il a été trouvé, selon la présente invention, qu'en greffant sur une chaîne siliconée un ou plusieurs groupements Benzimidazolyl-Benzazoles N-substitués ou de Benzofuranyl-Benzazoles, il était possible d'aboutir à de nouveaux composés présentant, outre des propriétés filtrantes excellentes dans le domaine des rayonnements UV-A et/ou UV-B, une très bonne solubilité dans les solvants organiques usuels et notamment les corps gras tels que les huiles, ainsi que d'excellentes propriétés cosmétiques, les rendant particulièrement appropriés à une utilisation comme filtres solaires dans des, ou pour la préparation de, compositions cosmétiques destinées à la protection de la peau et/ou des cheveux contre le rayonnement ultraviolet.

La présente invention a ainsi pour premier objet de nouveaux composés qui sont caractérisés par le fait qu'ils sont constitués d'une chaîne siliconée comportant au moins une unité de formule (1): 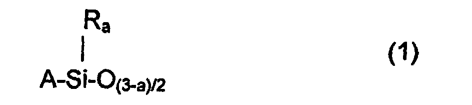 ou bien qu'ils sont des silanes répondant à la formule (2) suivante

A-SiR'₁R'₂R'₃ (2)

dans lesquelles :
- R désigne un groupe hydrocarboné saturé ou insaturé en C₁-C₃₀, un groupe hydrocarboné halogéné en C₁-C₈ ou un groupe triméthylsilyloxy,
- a est égal à 1 ou 2,
- R'₁, R'₂, R'₃, identiques ou différents, sont choisis parmi les radicaux alkyles et alcényles en C₁-C₈, linéaires ou ramifiés, ou un groupe triméthylsilyloxy,
- A est un radical de formule (I) suivante : 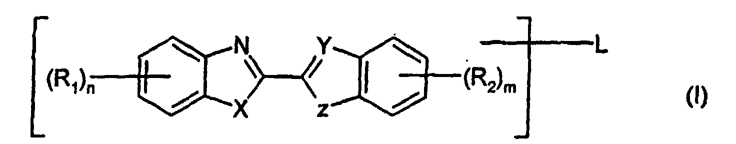 dans laquelle :
- L est un radical divalent permettant l'accrochage du radical A sur la chaîne siliconée ;
- les radicaux R₁ et R₂, identiques ou différents, représentent indépendamment un radical alkyle en C₁-C₁₀ linéaire ou ramifié ou un radical alcoxy en C₁-C₈ linéaire ou ramifié, deux R₁ adjacents ou deux R₂ adjacents pouvant former ensemble un groupement alkylidène dioxy dans lequel le groupe alkylidène contient de 1 à 2 atomes de carbone;
- X représente O, S, NH, NR₃ ou N-L;
- Y représente N ou CR₄ ;
- Z représente O, S, NH, NR₃ ou N-L;
- R₃ est un radical alkyle en C₁-C₁₀ linéaire ou ramifié, un radical aryle en C₆-C₁₂, un radical alcoxy carbonyle en C₁-C₁₀ ;
- R₄ est un atome d'hydrogène, un radical méthyle ou éthyle;
- n et m sont indépendamment 0, 1 ou 2;
étant entendu que l'accrochage de L peut se faire sur chacun des noyaux aromatiques ou sur X et/ou Z lorsque ces deux derniers sont NL et que dans le cas ou 2 groupements L existent, ces groupements L ne sont reliés qu'à des silanes de la formule (2).

De préférence, L répond à l'une des formules (a) ou (a^{·}') suivantes : 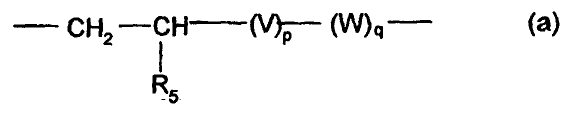 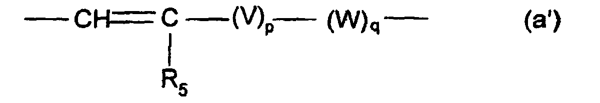 dans lesquelles :
- W représente O ou NH,
- V est un radical alcane di-yle en C₁-C₆ linéaire ou ramifié, éventuellement substitué par un radical hydroxyle ou alcoyle en C₂-C₈, linéaire ou ramifié,
- R₅ représente un atome d'hydrogène, un radical hydroxyle ou un radical alkyle en C₁-C₈, linéaire ou ramifié,
- p et q sont 0 ou 1 et obligatoirement q = 0 lorsque L est sur X ou Z.

De préférence encore, les composés selon l'invention répondent à l'une des formules (3) ou (4) suivantes : 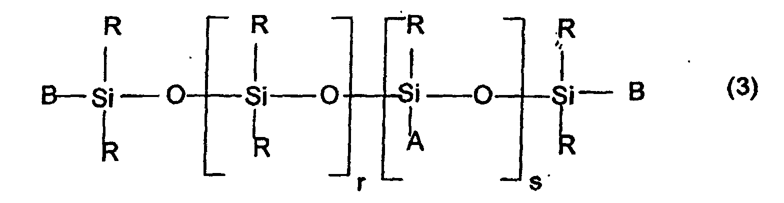 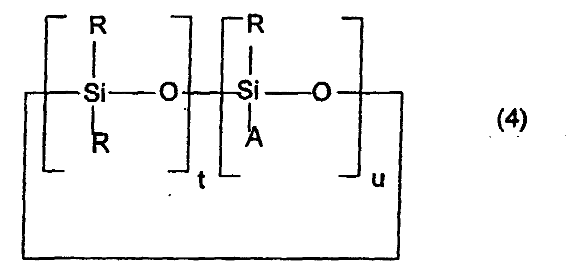 dans lesquelles :
- R désigne un groupe hydrocarboné saturé ou insaturé en C₁-C₃₀, un groupe hydrocarboné halogéné en C₁-C₈ ou un groupe triméthylsilyloxy,
- B, identiques ou différents, sont choisis parmi les radicaux R et le radical A,
- r est un nombre entier choisi entre 0 et 50 inclusivement,
- s est un nombre entier choisi entre 0 et 20 inclusivement et si s est 0, au moins un des deux symboles B est A,
- u est un nombre entier compris entre 1 et 6 inclus,
- t est un nombre entier entre 0 et 10 inclus,
- t + u est égal ou supérieur à 3.

Les composés de l'invention présentent une excellente liposolubilité et peuvent ainsi être utilisés à de grandes concentrations, ce qui confère aux compositions finales des indices de protection très élevés ; par ailleurs, ils se répartissent uniformément dans les supports cosmétiques classiques contenant au moins une phase grasse ou un solvant organique cosmétiquement acceptable et peuvent être ainsi appliqués sur la peau ou les cheveux pour constituer un film protecteur efficace.

En outre, les composés de l'invention présentent un excellent pouvoir filtrant intrinsèque à l'égard des rayonnements ultraviolet UV-A et/ou UV-B.

Ces nouveaux dérivés siliciés de Benzimidazolyl-Benzazoles N-substitués ou de Benzofuranyl-Benzazoles peuvent ainsi être utilisés comme filtres solaires pour la peau humaine et les cheveux. Ils peuvent aussi être utilisés comme agents protecteurs de la lumière dans l'industrie des plastiques.

De préférence, les radicaux R, identiques ou différents sont choisis parmi les radicaux alkyles en C₁-C₁₀, linéaires ou ramifiés, saturés ou insaturés, le radical phényle et le radical trifluoro-3,3,3 propyle, au moins 80% en nombre des radicaux R étant le radical méthyle.

Dans les formules (1) à (4) ci-dessus, on préfère plus particulièrement les dérivés statistiques ou bien définis à blocs présentant au moins l'une des caractéristiques suivantes :
- R est méthyle,
- B est méthyle,
- R₁ est méthoxy,
- R₂ est méthoxy,
- n est 0 ou 1,
- m est 0 ou 1,
- p est 1,
- q est 0,
- V est CH₂
- r est compris entre 0 et 3 inclus,
- s est compris entre 1 et 3 inclus,
- t + u est compris entre 3 et 5,
- R'₁, R'₂, R'₃ sont méthyle ou le groupement triméthylsilyloxy.

Parmi les composés siliconés préférentiels répondant à la formule (1) et plus particulièrement à la formule (3), on peut citer les composés suivants :
2-[1-[3-[1,3,3,3-tétraméthyl-1-[(triméthylsilyl)oxy]disiloxanyl]propyl]-1H-benzimidazol-2-yl]-benzoxazole
2-[1-[2-méthyl-3-[1,3,3,3-tétraméthyl-1-[(triméthylsilyl)oxy]disiloxanyl]propyl]-1H-benzimidazol-2-yl]-benzothiazole

Parmi les composés silaniques préférentiels répondant à la formule (2), on peut citer les composés suivants :
2-[1-(3-trimethylsilanyl-propyl)-1H-benzimidazol-2-yl]-benzoxazole,
6-méthoxy-1,1'-bis-(3-triméthylsilanyl-propyl)-1H,1'H-[2,2']bibenzimidazol
2-[1-(3-trimethylsilanyl-propyl)-1H-benzimidazol-2-yl]-benzothiazole

Pour préparer les dérivés de formules (1) à (4), on peut procéder classiquement en mettant en oeuvre une réaction d'hydrosilylation à partir du dérivé siloxanique ou silanique correspondant dans lequel, par exemple, tous les radicaux A sont des atomes d'hydrogène. Ce dérivé est dénommé par la suite dérivé à SiH.

Les groupes SiH peuvent être présents dans la chaîne et/ou aux extrémités de la chaîne. Ces dérivés à SiH sont des produits bien connus dans l'industrie des silicones et sont généralement disponibles dans le commerce. Ils sont par exemple décrits dans les brevets américains US-A-3220972, US-A-3697473 et US-A-4340709.

Les dérivés à SiH correspondant aux composés de formules (2), (3) et (4) peuvent être donc représentés par les formules (5) à (7) suivantes

H-SiR'₁R'₂R'₃ (5)

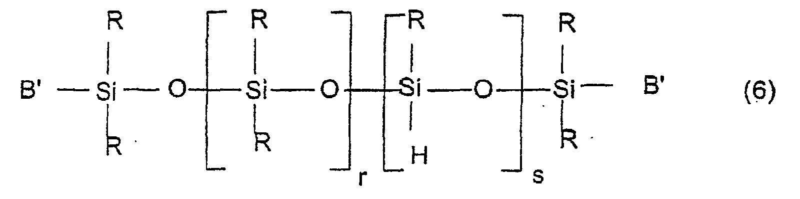

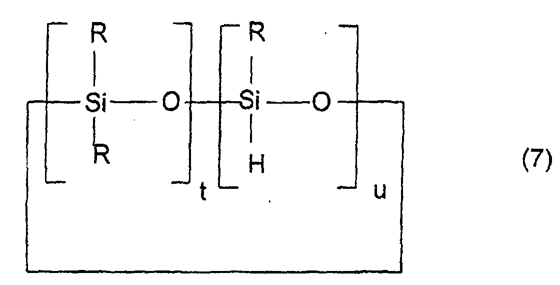

dans lesquelles :
- R'₁, R'₂ et R'₃ ont la signification donnée ci-dessus pour la formule (2),
- R, r, s, t et u ont la signification donnée ci-dessus pour les formules (3) et (4),
- B', identiques ou différents, sont choisis parmi les radicaux R et un atome d'hydrogène.

Afin de préparer les composés de l'invention de formules (2) à (4) ci-dessus, on procède de la manière suivante : sur le dérivé à SiH de formules (5), (6) ou (7), on effectue une réaction d'hydrosilylation en présence d'une quantité catalytiquement efficace d'un catalyseur au platine sur un dérivé organique de Benzimidazolyl-Benzazoles N-substitués ou de Benzofuranyl-Benzazoles choisi parmi ceux de formule (I') suivante : 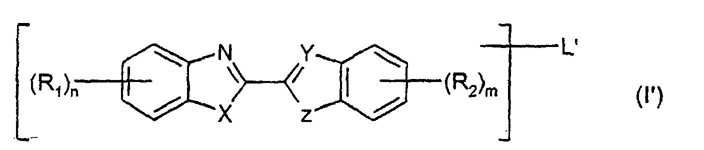 dans laquelle R₁, R₂, X, Y, Z, n et m ont la même signification qu'à la formule (I) ci-dessus et L'répond à l'une des deux formules (b) et (b') suivantes :

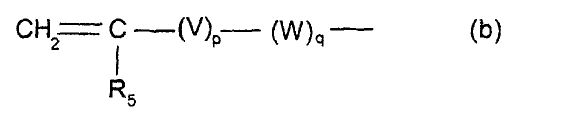

CH≡C―(V)ₚ―(W)_{q}― (b')

dans lesquelles W, R₅, V, p et q ont les mêmes significations qu'aux formules (a) et (a') ci-dessus.

La réaction d'hydrosilylation s'effectue donc selon l'une des deux réactions suivantes : 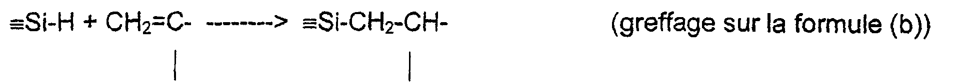 ou

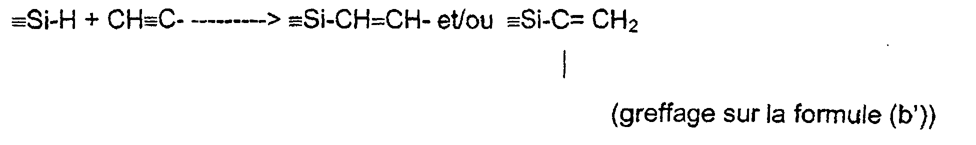

Comme dérivés de Benzimidazolyl-Benzazoles N-substitués ou de Benzofuranyl-Benzazoles utilisables pour la préparation des composés selon l'invention, on préfère tout particulièrement :
- 2-(1-allyl-1H-benzimidazol-2-yl)-benzoxazole
- 2-(1-allyl-1H-benzimidazol-2-yl)-benzothiazole
- 2-(1-méthallyl-1H-benzimidazol-2-yl)-benzothiazole

Les dérivés de formule (I') sont obtenus par condensation d'un halogénure d'alcène ou d'alcènyle sur un dérivé de formule (I") : 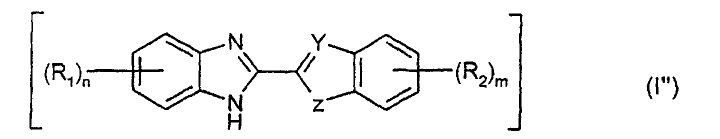 dans laquelle les radicaux R₁, R₂, Y, Z, n et m ont la même signification qu'aux formules (I) et (I'),

Comme dérivés de Benzimidazolyl-Benzazoles utilisables pour la préparation des composés selon l'invention de formule (I"), on préfère tout particulièrement :
- 2-(1H-benzimidazol-2-yl)-benzoxazole
- 2-(1H-benzimidazol-2-yl)-5-méthoxy-benzoxazole
- 2-(1H-benzimidazol-2-yl)-benzothiazole

Les dérivés de formule (I") peuvent être préparés selon les modes opératoires décrits dans le brevet EP 0843 995.

Les dérivés silaniques de formule (2) (A-Si-R'₁R'₂R'₃) conformes à l'invention, peuvent être obtenus selon un autre procédé de synthèse qui consiste à partir du dérivé de formule (I") ci-dessus, dans laquelle les radicaux R₁, R₂, Y, Z, n et m ont la même signification qu'aux formules (I) et (I') ci-dessus et à lui faire réagir un dérivé silanique de formule (8) suivante :

Hal-(Z)ₚ-CHR₅-CH₂-SiR'₁R'₂R'₃ (8)

dans laquelle Hal représente un halogène et plus particulièrement le chlore ou l'iode et les radicaux R₅, Z, R'₁, R'₂ R'₃ et p ont les mêmes significations que ci-dessus.

La présente invention a également pour objet une composition comprenant un composé de formule (1) ou (2) selon l'invention dans un support approprié. Le support peut être par exemple une composition de matière plastique. Il peut également être approprié pour une application topique. Dans ce cas, la composition selon l'invention est une composition cosmétique qui comprend un support cosmétiquement acceptable.

De préférence, la composition selon l'invention est une composition destinée à protéger une matière sensible au rayonnement ultraviolet, en particulier au rayonnement solaire, comprenant une quantité efficace d'au moins un composé conforme à l'invention. Dans une forme préférée de réalisation de l'invention, cette composition est destinée à protéger la peau et/ou les cheveux.

Les composés de formule (1) ou (2) sont généralement présents dans la composition de l'invention dans des proportions comprises entre 0,1 % et 20 % en poids, de préférence entre 0,5 % et 10 % en poids, par rapport au poids total de la composition.

Les compositions selon l'invention peuvent bien entendu contenir un ou plusieurs filtres solaires complémentaires actifs dans l'UVA et/ou l'UVB (absorbeurs), autres que les composés conformes à la présente invention, hydrophiles ou lipophiles. Ces filtres complémentaires peuvent être notamment choisis parmi les dérivés cinnamiques, les dérivés salicyliques, les dérivés du camphre, les dérivés de triazine, les dérivés de benzotriazole, les dérivés de la benzophénone, les dérivés du dibenzoylméthane, les dérivés de β,β-diphénylacrylate, les dérivés de l'acide p-aminobenzoïque, les polymères filtres et silicones filtres tels que ceux décrits dans la demande WO-93/04665.

Comme exemples de filtres solaires complémentaires actifs dans l'UV-A et/ou l'UV-B, on peut citer :
l'acide p-aminobenzoïque,
le p-aminobenzoate oxyéthyléné (25mol),
le p-diméthylaminobenzoate de 2-éthylhexyle,
le p-aminobenzoate d'éthyle N-oxypropyléné
le p-aminobenzoate de glycérol,
le salicylate d'homomenthyle,
le salicylate de 2-éthylhexyle,
le salicylate de triéthanolamine,
le salicylate de 4-isopropylbenzyle,
le 4-ter-butyl-4'-méthoxy-dibenzoylméthane,
le 4-isopropyl-dibenzoylméthane,
le 4-méthoxy cinnamate de 2-éthylhexyle,
le diisopropyl cinnamate de méthyle,
le 4-méthoxy cinnamate d'isoamyle,
le 4-méthoxy cinnamate de diéthanolamine,
l'anthranilate de menthyle,
le 2-éthylhexyl-2-cyano-3,3'-diphénylacrylate,
l'éthyl-2-cyano-3,3'-diphénylacrylate,
l'acide 2-phényl benzimidazole 5-sulfonique et ses sels,
le 3-(4'-triméthylammonium)-benzylidèn-bornan-2-on-méthylsulfate,
le 2-hydroxy-4-méthoxybenzophénone,
le 2-hydroxy-4-méthoxybenzophénone-5-sulfonate,
le 2,4-dihydroxybenzophénone,
le 2,2',4,4'-tétrahydroxybenzophénone,
le 2,2'-dihydroxy-4,4'diméthoxybenzophénone,
le 2-hydroxy-4-n-octoxybenzophénone,
le 2-hydroxy-4-méthoxy-4'-méthylbenzophénone,
l'acide urocanique,
l'acide α-(2-oxoborn-3-ylidène)-tolyl-4-sulfonique et ses sels
le 3-(4'-sulfo)benzylidèn-bornan-2-one et ses sels,
le 3-(4'méthylbenzylidène)-d,l-camphre,
le 3-benzylidène-d,l-camphre,
la 2,4,6-tris-[ p-(2'-éthylhexyl-1'-oxycarbonyl)anilino]-1,3,5-triazine,
la 2-[p-(tertiobutylamido)anilino]-4,6-bis-[(p-(2'-éthylhexyl-1'-oxycarbonyl)anilino]-1,3,5-triazine,
la 2,4-bis {[4-(2-éthyl-hexyloxy)-2-hydroxy]-phényl}-6-(4-méthoxy-phenyl)-1,3,5-triazine
l'acide 1,4-bis-benzimidazolyl-phènylèn-3, 3', 5, 5'-tétrasulfonique et ses sels,
le polymère de N-[(2 et 4)-[(2-oxoborn-3-ylidèn)méthyl], benzyl]-acrylamide,
le drométrizole trisiloxane (nom INCl),
les polyorganosiloxanes à fonction malonate.

Les compositions selon l'invention peuvent également contenir des agents de bronzage et/ou de brunissage artificiels de la peau (agents autobronzants), tels que par exemple de la dihydroxyacétone (DHA).

Les compositions selon l'invention peuvent encore contenir des pigments ou bien encore des nanopigments (taille moyenne des particules primaires : généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A-0518772 et EP-A- 0518773.

Elle peut contenir les adjuvants cosmétiques habituellement utilisés dans le domaine cosmétique, tels que des corps gras, des solvants organiques, de silicones, des épaississants, des adoucissants, les antioxydants, les opacifiants, les stabilisants, les émollients, les hydroxyacides, des agents anti-mousses, des agents hydratants, des parfums, des conservateurs, des tensio-actifs, des chargés, des séquestrants, des polymères anioniques, cationiques, non ioniques ou amphotères ou leurs mélanges, des propulseurs, des agents alcalinisants ou acidifiants, des colorants, ou tout autre ingrédient habituellement utilisé en cosmétique; en particulier pour la fabrication des compositions antisolaires.

Parmi les solvants organiques, on peut citer les alcools et polyols inférieurs tels que l'éthanol, l'isopropanol, le propylèneglycol, la glycérine et le sorbitol.

Les corps gras peuvent être constitués par une huile ou par une cire ou leurs mélanges, des acides gras, des esters d'acides gras, des alcools gras, la vaseline, la paraffine, la lanoline, la lanoline hydrogénée, la lanoline acétylée. Les huiles peuvent être choisies parmi les huiles animales, végétales, minérales ou de synthèse, et notamment l'huile de palme hydrogénée, l'huile de ricin hydrogénée, l'huile de vaseline, l'huile de paraffine, l'huile de Purcellin, les huiles de silicones, volatiles ou non, et les isoparaffines.

Bien entendu, l'homme de l'art veillera à choisir le ou les éventuels composés complémentaires cités ci-dessus et/ou leurs quantités de manière telle que les propriétés avantageuses attachées intrinsèquement au composé conforme à l'invention ne soient pas, ou substantiellement pas, altérées par la ou les adjonctions envisagées.

La composition cosmétique de l'invention peut être utilisée comme composition protectrice de l'épiderme humain ou des cheveux contre les rayons ultraviolets, comme composition antisolaire ou comme produit de maquillage.

Cette composition peut se présenter en particulier sous forme de lotion, de lotion épaissie, de gel, de crème, de lait, de poudre, de bâtonnet solide et éventuellement être conditionnée en aérosol et se présenter sous forme de mousse ou de spray.

Lorsque la composition cosmétique selon l'invention est plus particulièrement destinée à la protection de l'épiderme humain contre les rayons UV ou comme composition antisolaire, elle peut se présenter sous forme de suspension ou de dispersion dans des solvants ou des corps gras, ou encore sous forme d'émulsion (notamment de type H/E ou E/H, mais de préférence H/E) telle qu'une crème ou un lait, de dispersion vésiculaire, sous forme de pommade, de gel, de bâtonnet solide ou de mousse aérosol. Les émulsions peuvent contenir en outre des agents tensio-actifs anioniques, non ioniques, cationiques ou amphotères.

Lorsque la composition cosmétique selon l'invention est utilisée pour la protection des cheveux, elle peut se présenter sous forme de shampooing, de lotion, de gel ou composition à rincer, à appliquer avant ou après shampooing, avant ou après coloration ou décoloration, avant, pendant ou après permanente ou défrisage, de lotion ou gel coiffant ou traitant, de lotion ou gel pour le brushing ou la mise en plis, de laque pour cheveux, de composition de permanente ou de défrisage, de coloration ou décoloration des cheveux.

Lorsque la composition cosmétique selon l'invention est utilisée comme produit de maquillage des cils, des sourcils, des ongles, de la peau ou des cheveux, tels que crème de traitement de l'épiderme, fond de teint, bâton de rouge à lèvres, fards à paupière, fards à joues, ligneur encore appelé "eye-liner", mascara, gel colorant, elle peut se présenter sous forme solide ou pâteuse, anhydre ou aqueuse, comme des émulsions huile-dans-eau ou eau-dans-huile, des suspensions ou encore des gels.

L'invention a encore pour objet l'utilisation d'un composé conforme à l'invention dans des, ou pour la fabrication de, compositions destinées à protéger des matières sensibles au rayonnement ultraviolet, en particulier au rayonnement solaire.

L'invention a encore pour objet l'utilisation d'un composé de formule (1) ou (2) conforme à l'invention pour la préparation d'un médicament destiné à prévenir les effets néfastes des rayonnements UV.

L'invention a encore pour objet l'utilisation d'un composé de formule (1) ou (2), conforme à l'invention à titre d'agent filtrant les rayonnements UV, en particulier pour contrôler la couleur de la peau.

L'invention a enfin pour objet un procédé non thérapeutique de protection de la peau et/ou des cheveux contre le rayonnement ultraviolet, en particulier le rayonnement solaire, qui consiste à appliquer sur la peau ou les cheveux une quantité efficace de la composition cosmétique définie ci-dessus, ou d'un composé de formule (1) ou (2), tel que défini ci-avant.

L'invention a finalement pour objet un procédé non thérapeutique de contrôle de la variation de couleur de la peau due aux rayonnements UV, qui consiste à appliquer sur la peau une quantité efficace de la composition cosmétique définie ci-dessus, ou d'un composé de formule (1) ou (2), tel que défini ci-avant.

Les exemples qui suivent illustrent l'invention sans toutefois en limiter la portée.

### EXEMPLES DE PREPARATION

### EXEMPLE 1 : 2-[1-(3-trimethylsilanyl-propyl)-1H-benzimidazol-2-yl]-benzoxazole

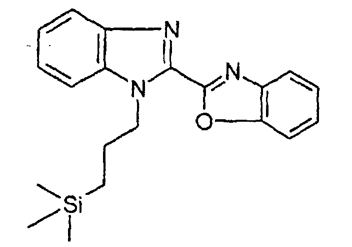

### a) Première étape : préparation du 2-trichlorométhyl-1H-benzimidazole :

A du 1,2-phénylène diamine (10,8 g, 0,1 mole) dissout dans 250 ml d'acide acétique à 15°C et sous agitation on ajoute goutte à goutte en 30 minutes du méthyltrichloroacetimidate (12,8 ml, 0,103 mole). On laisse 5 heures sous agitation à température ambiante. Le mélange réactionnel est versé dans de l'eau. Le précipité formé est filtré, lavé à l'eau et séché. On obtient 23,1 g (Rendement = 98 %) d' une poudre blanc cassé de 2-trichlorométhyl-1H-benzimidazole.

### b) Deuxième étape : préparation du 2-(1H-benzimidazol-2-yl)-benzoxazole:

A un mélange hétérogène du dérivé précédent (4,7 g, 0,02 mole) et de 2-aminophénol (2,4 g, 0,022 mole) agités à température ambiante dans 40 ml d'éthanol à 95%, on ajoute en 30 minutes 8,5 ml de triéthylamine (0,061 mole). Le mélange réactionnel est versé dans de l'eau. Le précipité formé est filtré, lavé à l'eau et séché. On obtient 4,7 g (Rendement = 100 %) d' une poudre beige de 2-(1H-benzimidazol-2-yl)-benzoxazole.

### c) Troisième étape : préparation du dérivé de l'exemple 1 :

A un mélange hétérogène du dérivé précédent (2 g, 8,5 mmole) et de carbonate de potassium (1,29 g) dans 10 ml de DMF chauffé à 55°C, on ajoute goutte-à-goutte en 15 minutes du 3-chloropropyltriméthylsilane (1,6 ml, 9,35 mmole). On poursuit le chauffage à 70°C pendant 9 heures. Le mélange réactionnel est refroidit et versé dans de l'eau. Le précipité obtenu est filtré, lavé à l'eau et séché. On obtient 2,6 g (Rendement : 87%) du dérivé de l'exemple 1 sous forme d'une poudre beige :

| | | |
|---|---|---|
| -UV (Ethanol) | λₘₐₓ = 328 nm, | εₘₐₓ = 34 900 |
| | λₘₐₓ = 345 nm, | εₘₐₓ = 23 660 |

### EXEMPLE 2 : 6-méthoxy-1,1'-bis-(3-triméthylsilanyl-propyl)-1H,1'H-[2,2']bibenzimidazole

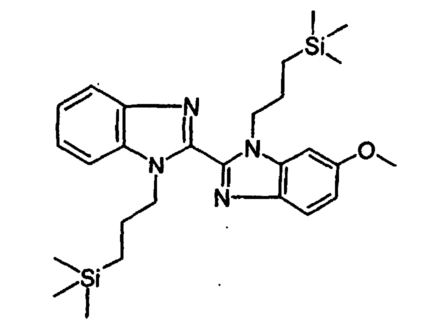

### a) Première étape : préparation du 6-méthoxy-1H,1'H-[2,2']bibenzimidazolyl:

A un mélange hétérogène de 2-trichlorométhyl-1H-benzimidazole (Première étape de l'exemple 1) (2,36 g, 0,01 mole) et de dichlorhydrate de 4-méthoxy-orthophénylène diamine (2,3 g, 0,011 mole) dans 20 ml d'éhanol à 95%, on ajoute goutte-à-goutte sous agitation à température ambiante en 50 minutes 7,1 ml de triéthylamine (0,051 mole). On laisse sous agitation à température ambiante pendant 2 heures 30 minutes. Le mélange réactionnel est versé dans de l'eau. Le précipité formé est filtré, lavé à l'eau et séché. Après chromatographie sur silice (éluant : dichlorométhane puis gradient dichlorométhane/méthanol), on obtient ainsi 1,4 g (Rendement : 53%) d'une poudre beige de 6-méthoxy-1H,1'H-[2,2']bibenzimidazolyl.

### b) Deuxième étape : préparation du dérivé de l'exemple 2 :

A un mélange hétérogène du dérivé précédent (1,28 g, 4,84 mmole) et de carbonate de potassium (1,47 g) dans 7 ml de DMF chauffé à 70°C, on ajoute goutte-à-goutte en 15 minutes du 3-chloropropyltriméthylsilane (1,61 ml, 10,65 mmole). On pousuit le chauffage à 85°C pendant 3 heures. Le mélange réactionnel est refroidit et versé dans de l'eau. La gomme obtenue est reprise dans le dichlorométhane. La phase organique est lavée à l'eau, séchée et le solvant évaporé. Après chromatographie sur silice (éluant heptane), on obtient ainsi 0,77 g (Rendement : 33%) du dérivé de l'exemple 2 sous forme d'une poudre blanc cassé :

| | | |
|---|---|---|
| -UV (Ethanol) | λₘₐₓ = 325 nm, | εₘₐₓ = 23 850 |
| | λₘₐₓ = 355 nm (épaulement), | εₘₐₓ = 16 550 |

### EXEMPLE 3 : 2-[1-[3-[1,3,3,3-tétraméthyl-1-[(triméthylsilyl)oxy]disiloxanyl]propyl]-1H-benzimidazol-2-yl]-benzoxazole

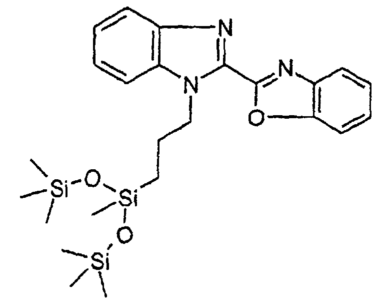

### a) Première étape : préparation du 2-(1-allyl-1H-benzimidazol-2-yl)-benzoxazole

A un mélange hétérogène de 2-(1H-benzimidazol-2-yl)-benzoxazole (Deuxième étape de l'exemple 1) (2 g, 8,5 mmole) et de carbonate de potassium (1,29g) dans 10 ml de DMF chauffé à 70°C, on ajoute goutte à goutte en 10 minutes du bromure d'allyle (1,13 ml, 9,35 mmole). On poursuit le chauffage à 70°C pendant 4 heures. Le mélange réactionnel est refroidit et versé dans de l'eau. Le précipité obtenu est filtré, lavé à l'eau et séché. On obtient 2,3 g (Rendement : 96%) de 2-(1-allyl-1H-benzimidazol-2-yl)-benzoxazole sous forme d'une poudre beige :

| | | |
|---|---|---|
| -UV (Ethanol) | λₘₐₓ = 327 nm, | εₘₐₓ = 34 100 |
| | λₘₐₓ = 344 nm, | εₘₐₓ = 23 400 |

### b) Deuxième étape : préparation du dérivé de l'exemple 3 :

A une solution du dérivé précédent (2 g, 7,26 mmole) et de catalyseur (complexe à 3-3.5% poids de Pt dans du cyclovinylméthylsiloxane de Hüls Petrarch PC085 : 200 µl) dans 8 ml de toluène sec porté à 80°C, on ajoute goutte-à-goutte en 20 minutes 1,62 g (7,26 mmole) d'heptaméthyltrisiloxane. On laisse à cette température pendant 3 heures. Un ajout supplémentaire de catalyseur au platine (200 µl) est nécessaire et on laisse sous agitation à 95°C pendant 8 heures. On concentre le mélange réactionnel et on obtient après chromatographie sur silice (éluant : heptane puis gradient heptane/EtOAc 95:5) 0,75 g (Rendement : 20%) du dérivé de l'exemple 3 sous forme d'une poudre jaune pâle:

| | | |
|---|---|---|
| -UV (Ethanol) | λₘₐₓ = 328 nm, | εₘₐₓ = 34 450 |
| | λₘₐₓ = 345 nm, | εₘₐₓ = 24 190 |

### EXEMPLE 4 : 2-[1-(3-trimethylsilanyl-propyl)-1H-benzimidazol-2-yl]-benzothiazole

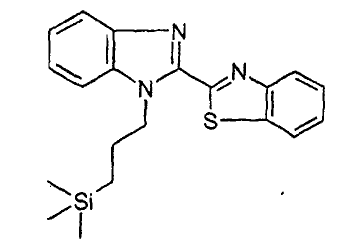

### a) Première étape : préparation du 2-(1H-benzoimidazol-2-yl)-benzothiazole

Le mélange hétérogène constitué de méthylbenzothiazole (59,6g, 0,4 mole), d'orthophénylène diamine (43,2g, 0,4 mole) et de soufre (38,4g, 1,2 mole) dans 300 ml de pyridine est chauffé au reflux de la pyridine pendant 22 heures. Le mélange réactionnel est refroidit et versé dans une solution de Brine. Après extraction avec de l'acétate d'éthyle, 3 lavages à l'eau de la phase organique, séchage sur sulfate de sodium et concentration du solvant sous vide, le solide obtenu est lavé à l'acétone. Après séchage on obtient le 2-(1H-benzoimidazol-2-yl)-benzothiazole (13,8g, Rendement 13%) sous forme d'un solide jaune.

### b) Deuxième étape : préparation du dérivé de l'exemple 4 :

A un mélange hétérogène du dérivé précédent (150 mg, 0,6 mmole) et de carbonate de potassium (90 mg) dans 10 ml de DMF chauffé à 55°C, on ajoute du 3-iodopropyltriméthylsilane (150 mg, 0,6 mmole). On poursuit le chauffage à 70°C pendant 1 heure. Le mélange réactionnel est refroidit et versé dans de l'eau. Après extraction à l'acétate d'éthyle et 3 lavages à l'eau de la phase organique, on isole après séchage et évaporation à sec 150 mg (Rendement : 68%) d'une huile jaune pâle qui cristallise pour donner le dérivé de l'exemple 4 sous forme d'une poudre jaune pâle :

| | | |
|---|---|---|
| -UV (Ethanol) | λₘₐₓ = 341 nm, | εₘₐₓ = 31 410 |
| | λₘₐₓ = 359 nm, | εₘₐₓ = 22 520 |

### EXEMPLE 5 : 2-[1-[2-méthyl-3-[1,3,3,3-tétraméthyl-1-[(triméthylsilyl)oxy]disiloxanyl]propyl]-1H-benzimidazol-2-yl]-benzothiazole

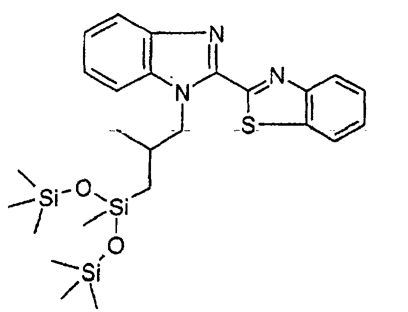

A un mélange hétérogène de 2-(1H-benzoimidazol-2-yl)-benzothiazole (Première étape de l'exemple 4) (4,2 g, 0,17 mole) et de carbonate de potassium (2,3 g) dans 50 ml de DMF chauffé à 60°C, on ajoute goutte à goutte en 10 minutes du 3-iodo-2-méthyl-3-[1,3,3,3-tétraméthyl-1-[(triméthylsilyl)oxy]disiloxanyl-propane (6,87 g, 0,017 mole). On poursuit le chauffage à 60°C pendant 4 heures. Le mélange réactionnel est refroidit et versé dans de l'eau. Après extraction à l'acétate d'éthyle et 3 lavages à l'eau de la phase organique, on isole après séchage et évaporation à sec 6,2 g (Rendement : 69%) de l'exemple 5 sous forme d'une huile jaune pâle:

| | | |
|---|---|---|
| -UV (Ethanol) | λₘₐₓ = 327 nm, | εₘₐₓ = 23 200 |
| | λₘₐₓ = 342 nm, | εₘₐₓ = 29 300 |
| | λₘₐₓ = 360 nm, | εₘₐₓ = 21 100 |

### EXEMPLES DE FORMULATION

| **COMPOSITION** | **EX A** |
|---|---|
| Mélange d'alcool cétylstéarylique et d'alcool cétylstéarylique oxyéthyléné (33 OE) 80/20 (SINNOWAX AO -HENKEL) | 7g |
| Mélange de mono et distéarate de glycérol (CERASYNT SD-V ISP) | 2g |
| Alcool cétylique | 1.5g |
| Polydiméthyl siloxane (DOW CORNING 200 FLUID -DOW CORNING) | 0.5g |
| Benzoate d'alcools en C₁₂/C₁₅ (WITCONOL TN -WITCO) | 10g |
| Composé de l'exemple 5 | 4g |
| Octocrylène (UVINUL N 539-BASF) | 10 |
| Oxyde de titane (MT-100 TV TAYCA) | 3g |
| Glycérine | 15g |
| Acide benzène 1,4-di(3-méthylidène-10-camphosulfonique) (MEXORYL SX-CHIMEX) | 1g |
| Triéthanolamine | 0.6g |
| Conservateurs | qs |
| Eau déminéralisée qsp | 100 g |

| **COMPOSITION** | **EX B** |
|---|---|
| Mélange mono /distéarate de glycerol / stéarate de polyéthylène glycol (100 OE) (ARLACEL 165 FL - ICI) | 2g |
| Alcool stéarylique (LANETTE 18 - HENKEL) | 1g |
| Acide stéarique d'huile de palme (STEARINE TP - STEARINERIE DUBOIS) | 2g |
| Poly diméthylsiloxane (DOW CORNING 200 FLUID - DOW CORNING) | 0.5g |
| Benzoate d'alcools en C₁₂/C₁₅ (WITCONOL TN -WITCO) | 15g |
| Triéthanolamine | 0.5g |
| Composé de l'exemple 5 | 2.5g |
| 4-ter-butyl-4'-méthoxy-dibenzoylméthane (PARSOL 1789-HOFFMANN-LAROCHE) | 2g |
| Octocrylène (UVINUL N539- BASF) | 8g |
| Oxyde de titane (TITANIUM DIOXYDE MT-100 TV TAYCA) | 3g |
| Propylène glycol | 4g |
| Glycérine | 4g |
| Acide benzène 1,4-di(3-méthylidène-10-camphosulfonique), (MEXORYL SX-CHIMEX) | 2.5g |
| Phosphate d'alcool hexadécylique,sel de potassium (AMPHISOL K - HOFFMAN LAROCHE) | 0.5g |
| Acide polyacrylique (SYNTHALEN K - 3V) | 0.3g |
| Hydroxypropyl méthyl cellulose (METHOCEL F4M -DOW CHEMICAL) | 0.15g |
| EDTA | 0.1g |
| Conservateurs | qs |
| Triéthanolamine | qs pH :7 |
| Eau démineralisée | 100 g |

## Revendications

1. Composé constitué d'une chaîne siliconée comportant au moins une unité de formule (1): 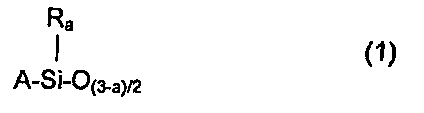 ou répondant à la formule (2) suivante :
A-SiR'₁R'₂R'₃ (2)
dans lesquelles :
- R désigne un groupe hydrocarboné saturé ou insaturé en C₁-C₃₀, un groupe hydrocarboné halogéné en C₁-C₈ ou un groupe triméthylsilyloxy,
- a est égal à 1 ou 2,
- R'₁, R₂', R'₃, identiques ou différents, sont choisis parmi les radicaux alkyles et alcényles en C₁-C₈, linéaires ou ramifiés, ou un groupe triméthylsilyloxy,
- A est un radical de formule (I) suivante : 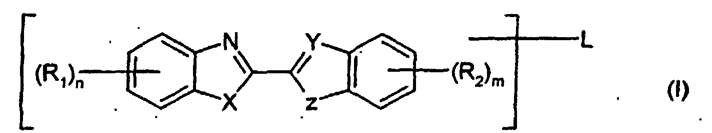 dans laquelle :
- L est un radical divalent permettant l'accrochage du radical A sur la chaîne siliconée ;
- les radicaux R₁ et R₂, identiques ou différents, représentent indépendamment un radical alkyle en C₁-C₁₀ linéaire ou ramifié ou un radical alcoxy en C₁-C₈ linéaire ou ramifié, deux R₁ adjacents ou deux R₂ adjacents pouvant former ensemble un groupement alkylidène dioxy dans lequel le groupe alkylidène contient de 1 à 2 atomes de carbone;
- X représente O, S, NH, NR₃ ou N-L;
- Y représente N ou CR₄ ;
- Z représente O, S, NH, NR₃ ou N-L;
- R₃ est un radical alkyle en C₁-C₁₀ linéaire ou ramifié, un radical aryle en C₆-C₁₂, un radical alcoxy carbonyle en C₁-C₁₀ ;
- R₄ est un atome d'hydrogène, un radical méthyle ou éthyle;
- n et m sont indépendamment 0, 1 ou 2 ;
étant entendu que l'accrochage de L peut se faire sur chacun des noyaux aromatiques ou sur X et/ou Z lorsque ces deux derniers sont N-L et que dans le cas ou 2 groupements L existent, ces groupements, L ne sont reliés qu'à des silanes de formule (2).

2. Composé selon la revendication 1, où L répond à l'une des formules (a) ou (a') suivantes : 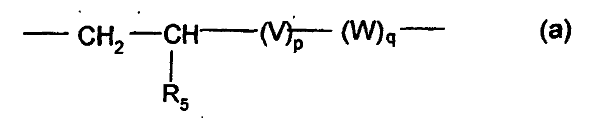 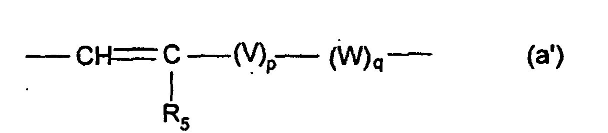 dans lesquelles :
- W représente O ou NH,
- V est un radical alcane di-yle en C₁-C₆ linéaire ou ramifié, éventuellement substitué par un radical hydroxyle ou alcoyle en C₂-C₈, linéaire ou ramifié,
- R₅ représente un atome d'hydrogène, un radical hydroxyle ou un radical alkyle en C₁-C₈, linéaire ou ramifié,
- p et q sont 0 ou 1 et obligatoirement q = 0 lorsque L est sur X ou Z.

3. Composé selon la revendication 1 ou 2, répondant à l'une des formules (3) ou (4) suivantés : 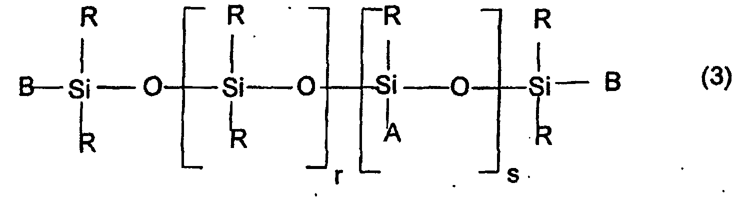 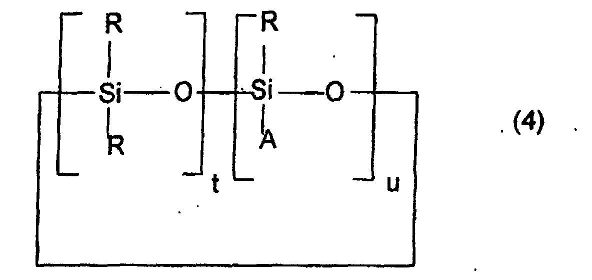 dans lesquelles :
- R désigne un groupe hydrocarboné saturé ou insaturé en C₁-C₃₀, un groupe hydrocarboné halogéné en C₁-C₈ ou un groupe triméthylsilyloxy,
- B, identiques ou différents, sont choisis parmi les radicaux R et le radical A,
- r est un nombre entier choisi entre 0 et 50 inclusivement,
- s est un nombre entier choisi entre 0 et 20 inclusivement et si s est 0, au moins un des deux symboles B est A,
- u est un nombre entier compris entre 1 et 6 inclus,
- t est un nombre entier entre 0 et 10 inclus,
- t + u est égal ou supérieur à 3.

4. Composé selon l'une quelconque des revendications 1 à 3, où dans la formule (1), (3) ou (4), les radicaux R, identiques ou différents sont choisis parmi les radicaux alkyles en C₁-C₁₀, linéaires ou ramifiés, le radical phényle et le radical trifluoro-3,3,3 propyle, au moins 80% en nombre des radicaux R étant le radical méthyle.

5. Composé selon l'une quelconque des revendications 1 à 4, où les composés répondant à l'une des formules (1) à (4) satisfait à au moins l'une des caractéristiques suivantes :
- R est méthyle,
- B est méthyle,
- R₁ est méthoxy,
- R₂ est méthoxy,
- n est 0 ou 1,
- m est 0 ou 1,
- p est 1,
- q est 0,
- V est CH₂
- r est compris entre 0 et 3 inclus,
- s est compris entre 1 et 3 inclus,
- t + u est compris entre 3 et 5,
- R'₁, R'₂, R'₃ sont méthyle ou le groupement triméthylsilyloxy.

6. Composé siliconé de formule (1) selon l'une quelconque des revendications 1 à 4, choisi dans le groupe constitué par :
2-[1-[3-[1,3,3,3-tétraméthyl-1-[(triméthylsilyl)oxy]disiloxanyl]propyl]-1H-benzimidazol-2-yl]-benzoxazole
2-[1-[3-[1,3,3,3-tétraméthyt-1-[(triméthylsilyl)oxy]disiloxanyl]propyl]-1H-benzimidazol-2-yl]-benzothiazole

7. Composé silanique de formule (2) selon l'une quelconque des revendications 1 à 4, choisi dans le groupe constitué par :
2-[1-(3-trimethylsilanyl-propyl)-1H-benzimidazol-2-yl]-benzoxazole
6-méthoxy-1,1'-bis-(3-triméthylsilanyl-propyl)-1H,1'H-[2,2']bibenzimidazolyle
2-[1-(3-trimethylsilanyl-propyl)-1H-benzimidazol-2-yl]-benzothiazole

8. Procédé de préparation des composés définis à l'une quelconque des revendications 1 à 7, **caractérisé par le fait qu'**il comprend l'étape suivante :
- on effectue, en présence d'une quantité catalytiquement efficace d'un catalyseur au platine, l'hydrosilylation d'un composé du type benzimidazolyl-Benzazole N-substitué ou du type benzofuranyl-Benzazole de formule (I') suivante : 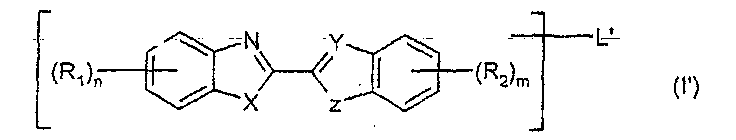 dans laquelle R₁, R₂, X, Y, Z, n et m ont la même signification qu'à la formule (I) définie dans la revendication 1 et L' répond à l'une des deux formules (b) et (b') suivantes : 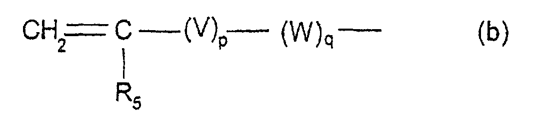
CH≡C―(V)ₚ―(W)_{q}― (b')
dans lesquelles W, R₅, V, p et q ont les mêmes significations définies dans l'une quelconque des revendications 1 et 2.
- par un dérivé SiH.

9. Procédé selon la revendication 8, **caractérisé par le fait que** le dérivé SiH est un composé répondant à l'une des formules (5) à (7) suivantes :
H-SiR'₁R'₂R'₃ (5) 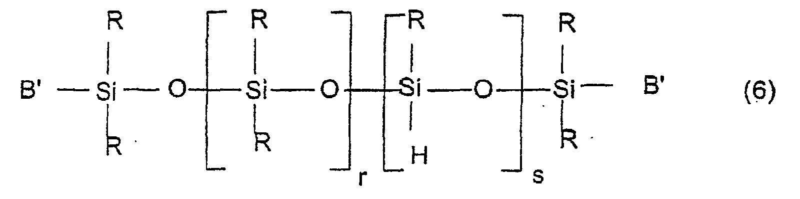 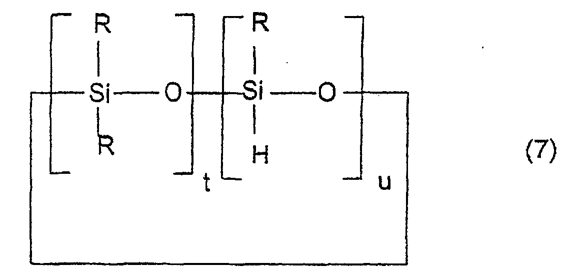
dans lesquelles
- R'₁, R'₂ et R'₃ ont la signification donnée à la revendication 1 pour la formule (2),
- R, r, s, t et u ont la signification donnée à la revendication 3 pour les formules (3) et (4),
- B', identiques ou différents, sont choisis parmi les radicaux R et un atome d'hydrogène.

10. Procédé selon la revendication 8 ou 9, **caractérisé par le fait que** le composé de formule (I') est obtenu par condensation d'un halogénure d'alcène ou d'alcènyle sur un dérivé de formule (I"): 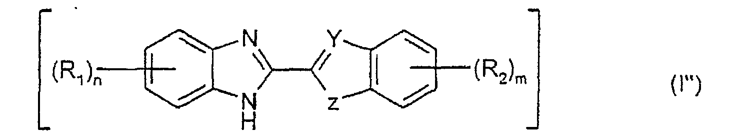 dans laquelle les radicaux R₁, R₂, Y, Z, n et m ont la même signification qu'aux formules (I) et (I').

11. Procédé de préparation des composés silaniques de formule (2) telles que définie dans les revendications 1 à 5 et 7, **caractérisé par le fait que** l'on fait réagir un composé de formule (I") telle que définie dans la revendication 10, avec un dérivé silanique de formule (8) suivante :
Hal-(Z)ₚ-CHR₅-CH₂-SiR'₁R'₂R'₃ (8)
dans laquelle Hal représente un halogène et plus particulièrement le chlore ou l'iode et les radicaux R₅, Z, R'₁, R'₂ R'₃ et p ont les mêmes significations que dans les revendications 1 et 2.

12. Composition de matière plastique comprenant au moins un composé de formule (1) ou (2) tel que défini dans l'une quelconque des revendications 1 à 7.

13. Composition cosmétique destinée à protéger la peau et/ou les cheveux du rayonnement UV comprenant dans un support cosmétiquement acceptable, au moins un composé de formule (1) ou (2) tel que défini dans l'une quelconque des revendications 1 à 7.

14. Composition selon l'une quelconque des revendications 12 et 13, **caractérisée par le fait que** le composé de formule (1) ou (2) est présent dans la composition à une teneur allant de 0,1 à 20 % en poids, par rapport au poids total de la composition, de préférence allant de 0,5 % à 10 % en poids, par rapport au poids total de la composition.

15. Composition selon l'une quelconque des revendications 13 à 14, **caractérisée par le fait que** ledit support cosmétiquement acceptable se présente sous la forme d'une émulsion de type huile-dans-eau.

16. Composition selon l'une quelconque des revendications 13 à 15, **caractérisée par le fait qu'**elle comprend en outre un ou plusieurs filtres organiques complémentaires actifs dans l'UV-A et/ou UV-B, hydrophiles ou lipophiles.

17. Composition selon la revendication 16 **caractérisée par le fait que** lesdits filtres organiques complémentaires sont choisis parmi les dérivés cinnamiques, les dérivés salicyliques, les dérivés du camphre, les dérivés de triazine, les dérivés de benzotriazole, les dérivés de la benzophénone, les dérivés du dibenzoylméthane, les dérivés de β,β-diphénylacrylate, les dérivés de l'acide p-aminobenzoïque, les polymères filtres et silicones filtres

18. Composition selon l'une quelconque des revendications 13 à 17, **caractérisée par le fait qu'**elle comprend en outre, à titre d'agents photoprotecteurs UV complémentaires, des pigments ou des nanopigments d'oxydes métalliques, enrobés ou non.

19. Composition selon la revendication 18, **caractérisée par le fait que** lesdits pigments ou nanopigments sont choisis parmi les oxydes de titane, de zinc, de fer, de zirconium, de cérium et leurs mélanges, enrobés ou non.

20. Composition selon l'une quelconque des revendications 13 à 19, **caractérisée par le fait qu'**elle comprend en outre au moins un agent de bronzage et/ou de brunissage artificiel de la peau.

21. Composition selon l'une quelconque des revendications 13 à 20, **caractérisée par le fait qu'**elle comprend en outre au moins un adjuvant choisi parmi les corps gras, les solvants organiques, les silicones, les épaississants, les adoucissants, les antioxydants, les opacifiants, les stabilisants, les émollients, les hydroxyacides, les agents anti-mousse, les agents hydratants, les vitamines, les parfums, les conservateurs, les tensioactifs, les charges, les séquestrants, les polymères anioniques, cationiques, non ioniques ou amphotères ou leurs mélanges, les propulseurs, les agents alcalinisants ou acidifiants, les colorants.

22. Composition selon l'une quelconque des revendications 13 à 21, **caractérisée par le fait qu'**elle se présente sous forme de lotion, lotion épaissie, de gel, de crème, de lait, de poudre, de bâtonnet solide, conditionnée en aérosol et se présenter sous forme de mousse ou de spray

23. Composition selon l'une quelconque des revendications 13 à 22, **caractérisée par le fait qu'**il s'agit d'une composition protectrice de l'épiderme humain ou d'une composition antisolaire et qu'elle se présente sous forme d'une dispersion vésiculaire non ionique, d'une émulsion, d'un gel, d'un gel crème, d'une suspension, d'une dispersion, d'une pommade, poudre, d'un bâtonnet solide, d'une mousse aérosol

24. Composition selon l'une quelconque des revendications 13 à 22, **caractérisée par le fait qu'**il s'agit d'une composition de maquillage des cils, dès sourcils, des ongles, de la peau ou des cheveux et qu'elle se présente sous forme solide ou pâteuse, anhydre ou aqueuse.

25. Composition selon l'une quelconque des revendications 13 à 22, **caractérisée par le fait qu'**il s'agit d'une composition destinée à la protection des cheveux contre les rayons ultraviolets et qu'elle se présente sous la forme d'un shampooing, d'une lotion, d'un gel ou composition à rincer, à appliquer avant ou après shampooing, avant ou après coloration ou décoloration, avant, pendant ou après permanente ou défrisage ; de lotion ou gel coiffant ou traitant ; de lotion ou gel pour le brushing ou la mise en plis ; de laque pour cheveux ; de composition de permanente ou de défrisage ; de composition de coloration ou décoloration des cheveux.

26. Utilisation d'au moins un composé de formule (1) ou (2) tel que défini dans l'une quelconque des revendications 1 à 7 pour la fabrication de compositions destinées à protéger des matières sensibles au rayonnement ultraviolet, en particulier au rayonnement solaire.

27. Utilisation d'au moins un composé de formule (1) ou (2) tel que défini dans l'une quelconque des revendications 1 à 7 pour la préparation d'un médicament destiné à prévenir les effets néfastes des rayonnements UV.

28. Utilisation d'un composé de formule (1) ou (2) tel que défini dans l'une quelconque des revendications 1 à 7 pour la fabrication d'une composition destinée à contrôler la variation de la couleur de la peau due aux rayonnement UV.

## Claims

1. Compound consisting of a silicone chain comprising at least one unit of formula (1): 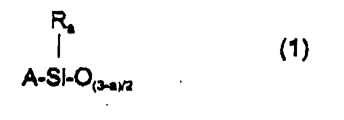 or corresponding to formula (2) below:
A-SiR'₁R'₂R'₃ (2)
in which:
- R denotes a saturated or unsaturated C₁-C₃₀ hydrocarbon-based group, a C₁-C₈ halohydrocarbon group or a trimethylsilyloxy group,
- a is equal to 1 or 2,
- R'₁, R'₂ and R'₃, which may be identical or different, are chosen from linear or branched C₁-C₈ alkyl and alkenyl radicals, or a trimethylsilyloxy group,
- A is a radical of formula (I) below: 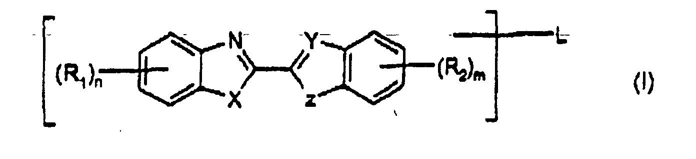 in which:
- L is a divalent radical for attaching the radical A to the silicone chain;
- the radicals R₁ and R₂, which may be identical or different, independently represent a linear or branched C₁-C₁₀ alkyl radical or a linear or branched C₁-C₈ alkoxy radical, in which two adjacent radicals R₁ or two adjacent radicals R₂ can together form an alkylidenedioxy group in which the alkylidene group contains 1 or 2 carbon atoms;
- X represents O, S, NH, NR₃ or N-L;
- Y represents N or CR₄;
- Z represents O, S, NH, NR₃ or N-L;
- R₃ is a linear or branched C₁-C₁₀ alkyl radical, a C₆-C₁₂ aryl radical or a C₁-C₁₀ alkoxycarbonyl radical;
- R₄ is a hydrogen atom or a methyl or ethyl radical;
- n and m are independently 0, 1 or 2;
it being understood that L can be attached to each of the aromatic nuclei or to X and/or Z when these two groups are N-L, and that when 2 groups L exist, these groups L are connected only to silanes of formula (2).

2. Compound according to Claim 1, in which L corresponds to either of formulae (a) and (a') below: 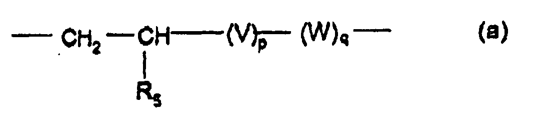 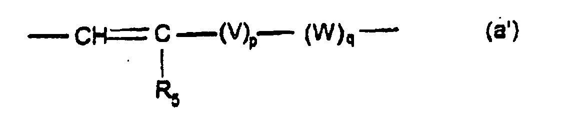 in which:
- W represents O or NH,
- V is a linear or branched C₁-C₆ alkanediyl radical optionally substituted with a hydroxyl radical or a linear or branched C₂-C₈ alkyl radical,
- R₅ represents a hydrogen atom, a hydroxyl radical or a linear or branched C₁-C₈ alkyl radical,
- p and q are 0 or 1 and, necessarily, q = 0 when L is on X or Z.

3. Compound according to Claim 1 or 2, corresponding to either of formulae (3) and (4) below: 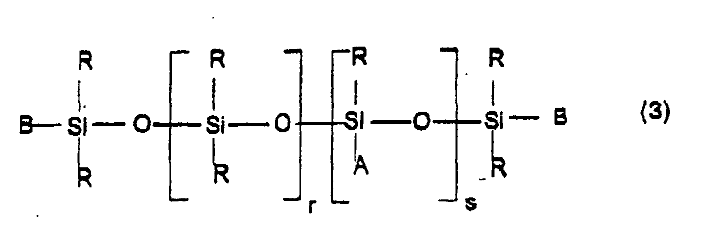 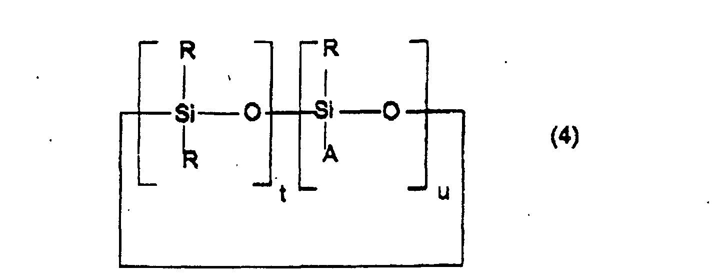 in which:
- R denotes a saturated or unsaturated C₁-C₃₀ hydrocarbon-based group, a C₁-C₈ halohydrocarbon group or a trimethylsilyloxy group,
- B, which may be identical or different, are chosen from the radicals R and the radical A,
- r is an integer chosen between 0 and 50 inclusive,
- s is an integer chosen between 0 and 20 inclusive, and if s is 0, at least one of the two symbols B is A,
- u is an integer between 1 and 6 inclusive,
- t is an integer between 0 and 10 inclusive,
- t + u is greater than or equal to 3.

4. Compound according to any one of Claims 1 to 3, in which, in formula (1), (3) or (4), the radicals R, which may be identical or different, are chosen from linear or branched C₁-C₁₀ alkyl radicals, the phenyl radical and the 3,3,3-trifluoropropyl radical, at least 80%, in numerical terms, of the radicals R being methyl radicals.

5. Compound according to any one of Claims 1 to 4, in which the compounds corresponding to one of formulae (1) to (4) satisfy at least one of the following characteristics:
- R is methyl,
- B is methyl,
- R₁ is methoxy,
- R₂ is methoxy,
- n is 0 or 1,
- m is 0 or 1,
- p is 1,
- q is 0,
- V is CH₂,
- r is between 0 and 3 inclusive,
- s is between 1 and 3 inclusive,
- t + u is between 3 and 5,
- R'₁, R'₂ and R'₃ are methyl or a trimethylsilyloxy group.

6. Silicone compound of formula (1) according to any one of Claims 1 to 4, chosen from the group consisting of:
2-[1-[3-[1,3,3,3-tetramethyl-1-[(trimethylsilyl)oxy]-disiloxanyl]propyl]-1H-benzimidazol-2-yl]benzoxazole
2-[1-[3-[1,3,3,3-tetramethyl-1-[(trimethylsilyl)oxy]-disiloxanyl]propyl]-1H-benzimidazol-2-yl]benzothiazole.

7. Silane compound of formula (2) according to any one of Claims 1 to 4, chosen from the group consisting of:
2-[1-(3-trimethylsilanylpropyl)-1H-benzimidazol-2-yl]-benzoxazole
6-methoxy-1,1'-bis(3-trimethylsilanylpropyl)-1H,1'H-[2,2']dibenzimidazolyle
2-[1-(3-trimethylsilanylpropyl)-lH-benzimidazol-2-yl]-benzothiazole.

8. Process for preparing the compounds defined in any one of Claims 1 to 7, **characterized in that** it comprises the following step:
- a hydrosilylation is carried out, in the presence of a catalytically effective amount of a platinum catalyst, on a compound of the N-substituted benzimidazolyl-benzazole or benzofuryl-benzazole type of formula (I') below: 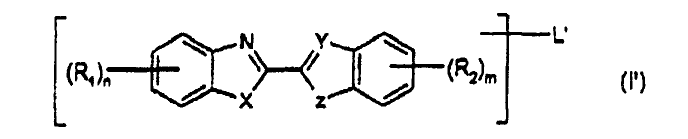 in which R₁, R₂, X, Y, Z, n and m have the same meaning as in formula (I) defined in Claim 1 and L' corresponds to either of the formulae (b) and (b') below: 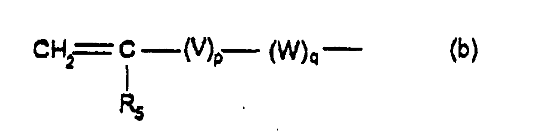
**CH≡C―(V)**_{**p**}**―(W)**_{**q**}**― (b')**
in which W, R₅, V, p and q have the same meanings defined in either of Claims 1 and 2,
- with a derivative containing SiH.

9. Process according to Claim 8, **characterized in that** the derivative containing SiH is a compound corresponding to one of the formulae (5) to (7) below:
**H-SiR'**_{**1**}**R'**_{**2**}**R'**_{**3**} **(5)** 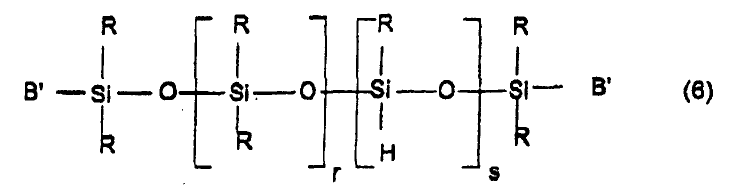 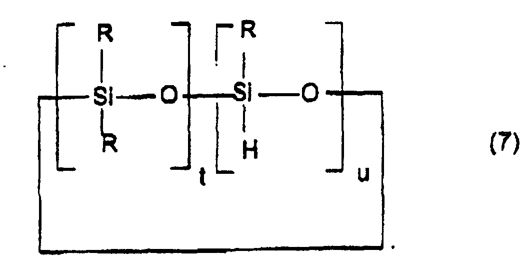
in which:
- R'₁, R'₂ and R'₃ have the meaning given in Claim 1 for formula (2),
- R, r, s, t and u have the meaning given in Claim 3 for formulae (3) and (4),
- B', which may be identical or different, are chosen from the radicals R and a hydrogen atom.

10. Process according to Claim 8 or 9, **characterized in that** the compound of formula (I') is obtained by coupling an alkene halide or an alkenyl halide with a derivative of formula (I"): 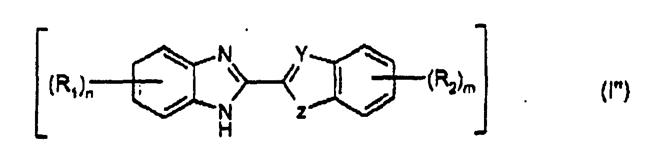 in which the radicals R₁, R₂, Y, Z, n and m have the same meaning as in formulae (I) and (I').

11. Process for preparing the silane compounds of formula (2) as defined in Claims 1 to 5 and 7, **characterized in that** a compound of formula (I") as defined in Claim 10 is reacted with a silane derivative of formula (8) below:
Hal-(Z)ₚ-CHR₅-CH₂-SiR'₁R'₂R'₃ (8)
in which Hal represents a halogen and more particularly chlorine or iodine, and the radicals R₅, Z, R'₁, R'₂, R'₃ and p have the same meanings as in Claims 1 and 2.

12. Plastic composition comprising at least one compound of formula (1) or (2) as defined in any one of Claims 1 to 7.

13. Cosmetic composition intended for protecting the skin and/or the hair against UV radiation, comprising, in a cosmetically acceptable support, at least one compound of formula (1) or (2) as defined in any one of Claims 1 to 7.

14. Composition according to either of Claims 12 and 13, **characterized in that** the compound of formula (1) or (2) is present in the composition in a content ranging from 0.1 to 20% by weight, relative to the total weight of the composition, preferably ranging from 0.5% to 10% by weight, relative to the total weight of the composition.

15. Composition according to either of Claims 13 and 14, **characterized in that** the said cosmetically acceptable support is in the form of an emulsion of oil-in-water type.

16. Composition according to any one of Claims 13 to 15, **characterized in that** it also comprises one or more additional hydrophilic or lipophilic organic screening agents that are active in the UV-A and/or UV-B range.

17. Composition according to Claim 16, **characterized in that** the said additional organic screening agents are chosen from cinnamic derivatives, salicylic derivatives, camphor derivatives, triazine derivatives, benzotriazole derivatives, benzophenone derivatives, dibenzoylmethane derivatives, β,β-diphenylacrylate derivatives, p-aminobenzoic acid derivatives, screening polymers and screening silicones.

18. Composition according to any one of Claims 13 to 17, **characterized in that** it also comprises, as additional UV-photoprotective agents, coated or uncoated metal oxide pigments or nanopigments.

19. Composition according to Claim 18, **characterized in that** the said pigments or nanopigments are chosen from titanium oxide, zinc oxide, iron oxide, zirconium oxide and cerium oxide and mixtures thereof, which may be coated or uncoated.

20. Composition according to any one of Claims 13 to 19, **characterized in that** it also comprises at least one agent for artificially tanning and/or browning the skin.

21. Composition according to any one of Claims 13 to 20, **characterized in that** it also comprises at least one adjuvant chosen from fatty substances, organic solvents, silicones, thickeners, softeners, antioxidants, opacifiers, stabilizers, emollients, hydroxy acids, antifoaming agents, moisturizers, vitamins, fragrances, preserving agents, surfactants, fillers, sequestering agents, anionic, cationic, nonionic or amphoteric polymers or mixtures thereof, propellants, acidifying or basifying agents, and dyes.

22. Composition according to any one of Claims 13 to 21, **characterized in that** it is in the form of a lotion, a thickened lotion, a gel, a cream, a milk, a powder or a solid tube, or is packaged as an aerosol and is in the form of a mousse or a spray.

23. Composition according to any one of Claims 13 to 22, **characterized in that** it is a composition for protecting the human epidermis or an antisun composition and **in that** it is in the form of a nonionic vesicular dispersion, an emulsion, a gel, a suspension, a dispersion, an ointment, a solid tube or an aerosol mousse.

24. Composition according to any one of Claims 13 to 22, **characterized in that** it is a composition for making up the eyelashes, the eyebrows, the nails, the skin or the hair and **in that** it is in solid or pasty, anhydrous or aqueous form.

25. Composition according to any one of Claims 13 to 22, **characterized in that** it is a composition intended for protecting the hair against ultraviolet rays and **in that** it is in the form of a shampoo, a lotion, a gel or a rinse-out composition, to be applied before or after shampooing, before or after dyeing or bleaching, or before, during or after permanent-waving or straightening the hair; a styling or medicated lotion or gel; a blow-drying or hairsetting lotion or gel; a hair lacquer; a permanent-waving or hair-straightening composition; a dyeing or bleaching composition for the hair.

26. Use of at least one compound of formula (1) or (2) as defined in any one of Claims 1 to 7, for the manufacture of compositions intended for protecting materials that are sensitive to ultraviolet radiation, in particular solar radiation.

27. Use of at least one compound of formula (1) or (2) as defined in any one of Claims 1 to 7, for the preparation of a medicinal product intended for preventing the harmful effects of UV radiation.

28. Use of a compound of formula (1) or (2) as defined in any one of Claims 1 to 7, for the manufacture of a composition intended for controlling the variation in the colour of the skin caused by UV radiation.

## Patentansprüche

1. Verbindung, die aus einer Siliconkette besteht, die mindestens eine Einheit der folgenden Formel (1) aufweist: 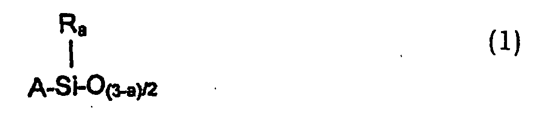 oder der folgenden Formel (2) entspricht:
A-SiR'₁R'₂R'₃ (2),
worin:
- R eine gesättigte oder ungesättigte C₁₋₃₀-Kohlenwasserstoffgruppe, eine halogenierte C₁₋₈-Kohlenwasserstoffgruppe oder eine Trimethylsilyloxygruppe bedeutet,
- a 1 oder 2 ist,
- die Gruppen R'₁, R'₂ und R'₃, die identisch oder voneinander verschieden sind, unter den geradkettigen oder verzweigten Alkyloder Alkenylgruppen mit 1 bis 8 Kohlenstoffatomen oder Trimethylsilyloxy ausgewählt sind,
- A eine Gruppe der folgenden Formel (I) ist: 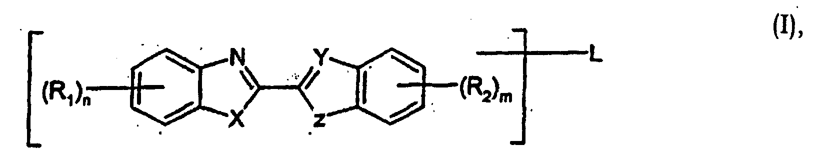
worin bedeuten:
- L eine zweiwertige Gruppe, über die die Gruppe A an die Siliconkette gebunden werden kann,
- die Gruppen R₁ und R₂, die identisch oder voneinander verschieden sind, unabhängig voneinander eine geradkettige oder verzweigte C₁₋₁₀-Alkylgruppe oder eine geradkettige oder verzweigte C₁₋₈-Alkoxygruppe, wobei zwei angrenzende Gruppen R₁ oder zwei angrenzende R₂ gemeinsam eine Alkylidendioxygruppe bilden können, wobei die Alkylidengruppe 1 bis 2 Kohlenstoffatome aufweist,
- X O, S, NH, NR₃ oder N-L,
- Y N oder CR₄,
- Z O, S, NH, NR₃ oder N-L,
- R₃ eine geradkettige oder verzweigte C₁₋₁₀-Alkylgruppe, eine C₆-C₁₂-Arylgruppe oder eine C₁₋₁₀-Alkoxycarbonylgruppe,
- R₄ Wasserstoff, Methyl oder Ethyl,
- n und m unabhängig voneinander 0, 1 oder 2,
- mit der Maßgabe, daß die Bindung von L an jedem aromatischen Ring oder an X und/oder Z erfolgen kann, wenn diese NL bedeuten, und daß, wenn 2 Gruppen L vorliegen, diese Gruppen L nur an die Silane der Formel (2) gebunden sind.

2. Verbindung nach Anspruch 1, worin die Gruppe L vorzugsweise einer der folgenden Formeln (a) oder (a') entspricht: 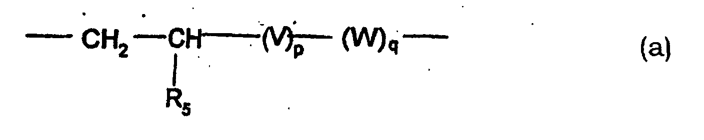 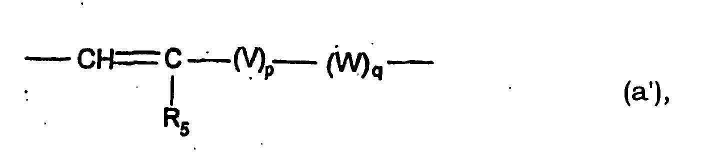 worin bedeuten:
- W O oder NH,
- V eine geradkettige oder verzweigte C₁₋₆-Alkandiylgruppe, die gegebenenfalls mit einer Hydroxygruppe oder einer geradkettigen oder verzweigten C₂₋₈-Alkylgruppe substituiert ist,
- R₅ Wasserstoff, Hydroxy oder eine geradkettige oder verzweigte C₁₋₈-Alkylgruppe, und
- p und q 0 oder 1 und zwingend q = 0, wenn sich L an X oder Z befindet.

3. Verbindung nach Anspruch 1 oder 2, die einer der folgenden Formeln (3) oder (4) entspricht: 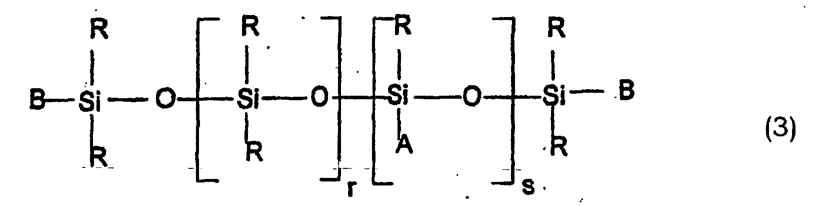 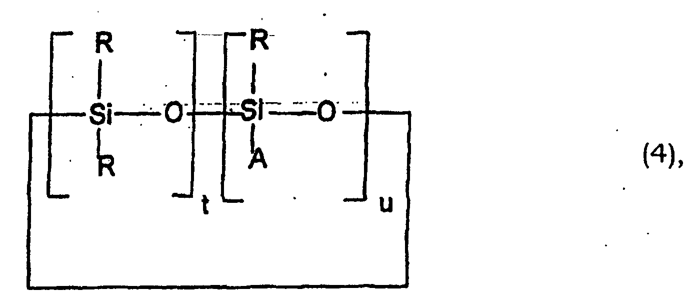 worin:
· die Gruppen R eine gesättigte oder ungesättigte C₁₋₃₀-Kohlenwasserstoffgruppe, eine halogenierte C₁₋₈-Kohlenwasserstoffgruppe oder eine Trimethylsilyloxygruppe bedeuten,
· die Gruppen B, die identisch oder voneinander verschieden sind, unter den Gruppen R und der Gruppe A ausgewählt sind,
· r Null oder eine ganze Zahl im Bereich von 1 bis 50 bedeutet,
· s Null oder eine ganze Zahl im Bereich 1 bis 20 bedeutet, wobei mindestens eine der beiden Gruppen B A bedeutet, wenn s Null ist,
· u eine ganze Zahl im Bereich von 1 bis 6 bedeutet,
· t Null oder eine ganze Zahl im Bereich von 1 bis 10 bedeutet, und
· t + u mindestens 3 ist.

4. Verbindung nach einem der Ansprüche 1 bis 3, wobei in der Formel (1), (3) oder (4) die Gruppen R, die identisch oder voneinander verschieden sind, unter den geradkettigen oder verzweigten C₁₋₁₀-Alkylgruppen, Phenyl und 3,3,3-Trifluorpropyl ausgewählt sind, wobei mindestens 80 % der Gruppen R Methyl bedeuten.

5. Verbindung nach einem der Ansprüche 1 bis 4, **dadurch gekennzeichnet, daß** sie einer der Formeln (1) bis (4) entspricht, für die mindestens eine der folgenden Bedingungen erfüllt ist:
- R ist Methyl;
- B ist Methyl;
- R₁ ist Methoxy;
- R2 ist Methoxy;
- n ist 0 oder 1;
- m ist 0 oder 1;
- p ist 1;
- q ist 0;
- V ist CH₂;
- r liegt im Bereich von 0 bis 3;
- s liegt im Bereich von 1 bis 3;
- t + u liegt im Bereich von 3 bis 5;
- R'₁, R'₂ und R'₃ bedeuten Methyl oder Trimethylsilyloxy.

6. Siliconverbindung der Formel (1) nach einem der Ansprüche 1 bis 4, die unter den folgenden Verbindungen ausgewählt ist:
2-[1-[3-[1,3,3,3-Tetramethyl-1-[(trimethylsilyl)oxy]disiloxanyl]-propyl]-1H-benzimidazol-2-yl]-benzoxazol, und
2-[1-[3-[1,3,3,3-Tetramethyl-1-[(trimethylsilyl)oxy]disiloxanyl]-propyl]-1H-benzmidazol-2-yl]-benzothiazol.

7. Silanverbindung der Formel (2) nach einem der Ansprüche 1 bis 4, die unter den folgenden Verbindungen ausgewählt ist:
2-[1-(3-Trimethylsilanyl-propyl)-1H-benzimidazol-2-yl]-benzoxazol, 6-Methoxy-1,1'-bis(3-trimethylsilanyl-propyl]-1H,1'H-[2,2']dibenzimidazol und
2-[1-(3-Trimethylsilanyl-propyl]-1H-benzimidazol-2-yl]-benzothiazol.

8. Verfahren zur Herstellung der Verbindungen nach einem der Ansprüche 1 bis 7, **gekennzeichnet durch** den folgenden Schritt:
- in Gegenwart einer katalytisch wirksamen Menge eines Platinkatalysators wird eine Hydrosilylierungsreaktion mit einem SiH-Derivat und einer Verbindung vom Typ N-substituiertes Benzimidazolyl-benzazolderivat oder vom Typ Benzofuranylbenzazolderivat der folgenden Formel (I') durchgeführt: 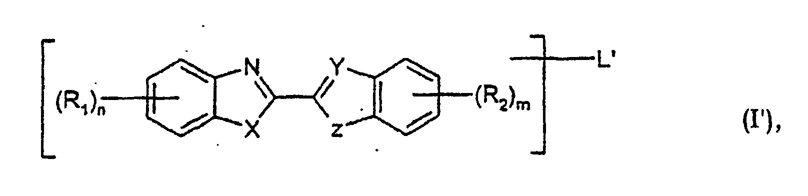 worin R₁, R₂, X, Y, Z, n und m die für die Formel (I) in Anspruch 1 angegebenen Bedeutungen aufweisen und worin L' einer der beiden folgenden Formeln (b) und (b') entspricht: 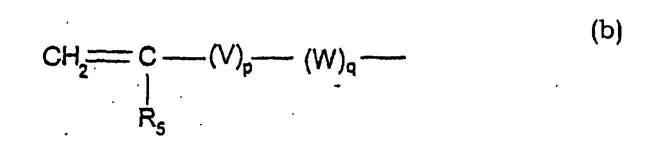
CH≡C―(V)ₚ―(W)_{q}― (b'),
worin die Gruppen W, R₅, V, p und q die oben in den Ansprüchen 1 und 2 angegebenen Bedeutungen aufweisen.

9. Verfahren nach Anspruch 8, **dadurch gekennzeichnet, daß** das SiH-Derivat eine Verbindung ist, die einer der folgenden Formeln (5) bis (7) entspricht:
H-SiR'₁R'₂R'₃ (5) 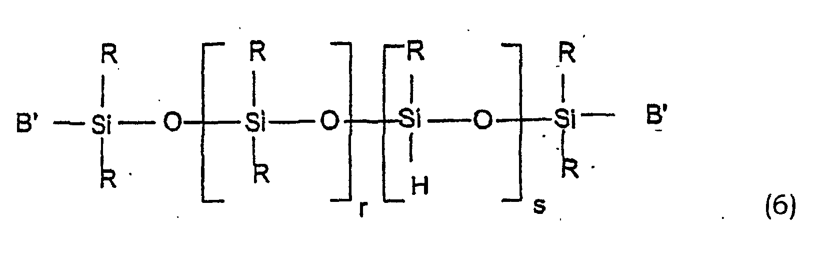 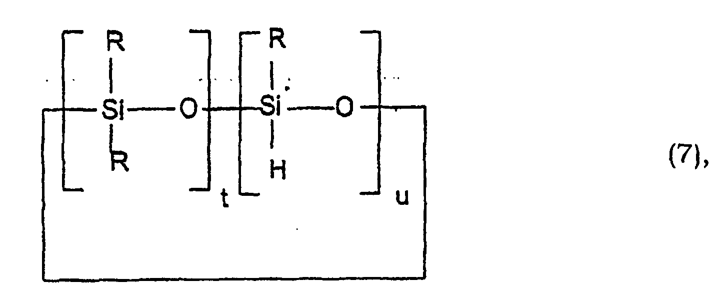
worin:
- R'₁, R'₂ und R'₃ die in Anspruch 1 für die Formel (2) angegebenen Bedeutungen aufweisen,
- R, r, s, t und u die in Anspruch 3 für die Formeln (3) und (4) angegebenen Bedeutungen aufweisen, und
- die Gruppen B', die identisch oder voneinander verschieden sind, unter den Gruppen R und Wasserstoff ausgewählt sind.

10. Verfahren nach Anspruch 8 oder 9, **dadurch gekennzeichnet, daß** die Verbindung der Formel (I') durch Kondensation eines Alkenoder Alkenylhalogenids mit einem Derivat der Formel (II") hergestellt wird: 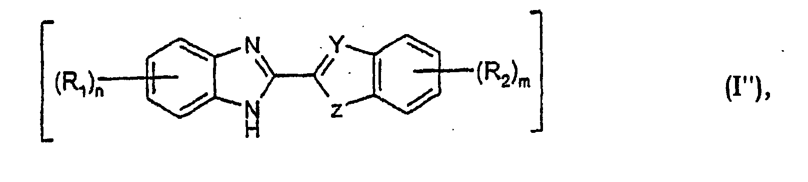 worin die Gruppen R₁, R₂, Y, Z, n und m die für die Formeln (I) und (I') angegebenen Bedeutungen aufweisen.

11. Verfahren zur Herstellung von Silanverbindungen der Formel (2) nach den Ansprüchen 1 bis 5 und 7, **dadurch gekennzeichnet, daß** eine Verbindung der Formel (I") nach Anspruch 10 mit einem Silanderivat der folgenden Formel (8) umgesetzt wird:
Hal-(Z)ₚ-CHR₅-CH₂-Si-R'₁R'₂R'₃ (8),
worin Hal Halogen und insbesondere Chlor oder Iod bedeutet und R₅, Z, R'₁, R'₂, R'₃ und p die oben in Anspruch 1 und 2 genannten Bedeutungen aufweisen.

12. Zusammensetzung aus Kunststoff, die mindestens eine Verbindung der Formel (1) oder (2) nach einem der Ansprüche 1 bis 7 enthält.

13. Kosmetische Zusammensetzung, die zum Schutz der Haut und/oder der Haare gegen UV-Strahlung vorgesehen ist und die in einem kosmetisch akzeptablen Träger mindestens eine Verbindung der Formel (1) oder (2) nach einem der Ansprüche 1 bis 7 enthält.

14. Zusammensetzung nach einem der Ansprüche 12 und 13, **dadurch gekennzeichnet, daß** die Verbindung der Formel (1) oder (2) in der Zusammensetzung in einem Mengenanteil von 0,1 bis 20 Gew.-%, bezogen auf das Gesamtgewicht der Zusammensetzung, und vorzugsweise im Bereich von 0,5 bis 10 Gew.-%, bezogen auf das Gesamtgewicht der Zusammensetzung, vorliegt.

15. Zusammensetzung nach einem der Ansprüche. 13 bis 14, **dadurch gekennzeichnet, daß** der kosmetisch akzeptable Träger in Form einer Emulsion vom Öl-in-Wasser-Typ vorliegt.

16. Zusammensetzung nach einem der Ansprüche 13 bis 15, **dadurch gekennzeichnet, daß** sie ferner ein oder mehrere zusätzliche, hydrophile oder lipophile, im UV-A- oder UV-B-Bereich wirksame organische Filter enthält.

17. Zusammensetzung nach Anspruch 16, **dadurch gekennzeichnet, daß** die zusätzlichen Filter unter den Zimtsäurederivaten, Salicylsäurederivaten, Campherderivaten, Triazinderivaten, Benzotriazolderivaten, Benzophenonderivaten, Dibenzoylmethanderivaten, β,β-Diphenylacrylatderivaten, p-Aminobenzoesäurederivaten und den Filterpolymeren und Filtersiliconen ausgewählt sind.

18. Zusammensetzung nach einem der Ansprüche 13 bis 17, **dadurch gekennzeichnet, daß** sie als zusätzliche UV-Lichtschutzmittel ferner Pigmente oder Nanopigmente von Metalloxiden, die gegebenenfalls umhüllt sind, enthält.

19. Zusammensetzung nach Anspruch 18, **dadurch gekennzeichnet, daß** die umhüllten oder nicht umhüllten Pigmente oder Nanopigmente unter den Oxiden von Titan, Zink, Eisen, Zirconium oder Cer oder deren Gemischen ausgewählt sind.

20. Zusammensetzung nach einem der Ansprüche 13 bis 19, **dadurch gekennzeichnet, daß** sie ferner mindestens ein Mittel zum Braunfärben und/oder zur künstlichen Braufärbung der Haut enthält.

21. Zusammensetzung nach einem der Ansprüche 13 bis 20, **dadurch gekennzeichnet, daß** sie ferner einen Zusatzstoff enthält, der ausgewählt ist unter: Fettsubstanzen, organischen Lösungsmitteln, Siliconen, Verdickungsmitteln, reizlindernden Mitteln, Antioxidantien, Trübungsmitteln, Stabilisatoren, Emollientien, Hydroxysäuren, Antischaummitteln, Hydratisierungsmitteln, Vitaminen, Parfums, Konservierungsmitteln, grenzflächenaktiven Stoffen, Füllstoffen, Maskierungsmitteln, anionischen, kationischen, nichtionischen oder amphoteren Polymeren oder deren Gemischen, Treibmitteln, Mitteln zum Ansäuern oder Alkalischmachen und Farbmitteln.

22. Zusammensetzung nach einem der Ansprüche 13 bis 21, **dadurch gekennzeichnet, daß** sie als Lotion, dickflüssige Lotion, Gel, Creme, Milch, Puder, fester Stift oder als Aerosol konfektioniert als Schaum oder Spray vorliegt.

23. Zusammensetzung nach einem der Ansprüche 13 bis 22, **dadurch gekennzeichnet, daß** es sich um eine Zusammensetzung zum Schutz der menschlichen Epidermis oder ein Sonnenschutzmittel handelt und dadurch, daß sie als nichtionische Vesikeldispersion, Emulsion, Gel, Suspension, Dispersion, Pomade, fester Stift oder Aerosolschaum vorliegt.

24. Zusammensetzung nach einem der Ansprüche 13 bis 22, **dadurch gekennzeichnet, daß** es sich um eine Zusammensetzung zum Schminken der Wimpern, der Augenbrauen, der Nägel, der Haut oder der Haare handelt und dadurch, daß sie in fester oder pastöser, wasserfreier oder wässeriger Form vorliegt.

25. Zusammensetzung nach einem der Ansprüche 13 bis 22, **dadurch gekennzeichnet, daß** es sich um eine Zusammensetzung zum Schutz der Haare gegen UV-Strahlung handelt und dadurch, daß sie als Haarwaschmittel, Lotion, Gel, Zusammensetzung zum Ausspülen, zur Anwendung vor oder nach der Haarwäsche, zur Anwendung vor oder nach der Färbung oder Entfärbung und zur Anwendung vor, während oder nach einer permanenten Verformung oder Haarglättung, als Frisier- oder Behandlungslotion, Frisieroder Behandlungsgel, Lotion oder Gel zum Fönen oder für Wasserwellen, als Haarlack, als Zusammensetzung zur Dauerwellverformung oder Haarglättung und als Zusammensetzung zum Färben oder Entfärben der Haare vorliegt.

26. Verwendung mindestens einer Verbindung der Formel (1) oder (2) nach einem der Ansprüche 1 bis 7 zur Herstellung von Zusammensetzungen, die dazu dienen sollen, gegenüber UV-Strahlung und insbesondere gegenüber Sonnenlicht empfindliche Materialien zu schützen.

27. Verwendung mindestens einer Verbindung der Formel (1) oder (2) nach einem der Ansprüche 1 bis 7 zur Herstellung eines Arzneimittels, das den schädlichen Wirkungen der UV-Strahlung vorbeugen soll.

28. Verwendung mindestens einer Verbindung der Formel (1) oder (2) nach einem der Ansprüche bis 7 zur Herstellung von Zusammensetzungen, die dazu dienen sollen, die von der UV-Strahlung bewirkte Farbänderung der Haut zu kontrollieren.
